# FASCICULE DE BREVET EUROPEEN

(11) **EP 0 912 634 B1**
(45) Date de publication et mention de la délivrance du brevet: **06.11.2002**
(21) Numéro de dépôt: 97933723.5
(22) Date de dépôt: 11.07.1997
(51) Int. Cl.: C08L 1/02, A23L 1/00, A61K 7/00, C11D 3/00, C04B 24/00

(54) **ADDITIVATION DE NANOFIBRILLES DE CELLULOSE ESSENTIELLEMENT AMORPHES AVEC DE LA CELLULOSE CARBOXYLEE A HAUT DEGRE DE SUBSTITUTION**
ZUSAMMENSETZUNG VON NANOFIBRILLEN VON CELLULOSE MIT CARBOXYLIERTEM CELLULOSE MIT HOHEM SUBSTITUTIONSGRAD
ADDITIVATION OF ESSENTIALLY AMORPHOUS CELLULOSE NANOFIBRILS WITH CARBOXYL CELLULOSE WITH A HIGH DEGREE OF SUBSTITUTION

(30) Priorité: 15.07.1996 FR 9609062; 27.09.1996 FR 9611779
(43) Date de publication de la demande: 06.05.1999
(60) Demande divisionnaire: 00202426.3
(73) Titulaire: RHODIA CHIMIE, 92408 Courbevoie Cédex (FR)
(72) Inventeur: CANTIANI, Robert, F-92800 Puteaux (FR); GUERIN, Gilles, F-95600 Eaubonne (FR); SENECHAL, Alain, F-94220 Charenton (FR); VINCENT, Isabelle, F-27000 Evreux (FR); BENCHIMOL, Jo[l, F-27800 Francqueville (FR)
(74) Mandataire: Dubruc, Philippe
(86) Numéro de dépôt international: FR9701291
(87) Numéro de publication internationale: WO98002487

(56) Documents cités:
- EP-A- 0 102 829
- EP-A- 0 120 471
- EP-A- 0 198 094
- EP-A- 0 537 554
- EP-A- 0 726 356
- WO-A-95/02966
- US-A- 4 659 388

## Description

La présente invention a pour objet des compositions comprenant des nanofibrilles de cellulose essentiellement amorphes, au moins un additif choisi parmi la cellulose carboxylée présentant un degré de substitution supérieur à 0,95, un polysaccharide naturel, un polyol, et éventuellement au moins un co-additif, ainsi que leur procédé de préparation.

Elle concerne des suspensions obtenues à partir de telles compositions.

Les microfibrilles et les nanofibrilles de cellulose sont des composés bien connus qui trouvent leurs utilisations comme additif modifiant la texture des milieux dans lesquels elles sont introduites. Dans le cas des milieux fluides, elles modifient leur viscosité voire leur profil rhéologique.

Cependant, il existe un problème avec les microfibrilles et les nanofibrilles de cellulose. En effet, elles sont obtenues sous la forme d'une suspension aqueuse, dont la teneur en matières sèches est relativement faible, de l'ordre de 1 à 5 % en poids environ. Le développement de ces produits sous une telle présentation n'est donc pas rentable économiquement, tant sur le plan du stockage que du transport, par exemple. On a donc pensé de manière évidente à les présenter sous une forme sèche. Malheureusement, lorsque les suspensions de microfibrilles ou de nanofibrilles de cellulose sont séchées, il se crée des liaisons hydrogène très fortes entre les fibrilles qui rendent nécessaires la mise en oeuvre de moyens très cisaillants pour redisperser ces fibrilles, lorsqu'il est possible de les remettre en suspension.

On a tenté de proposer des solutions au problème de séchage des microfibrilles de cellulose. Ainsi, des additifs ont été introduits lors de la préparation de suspensions de microfibrilles, et plus particulièrement au moment de l'homogénéisation.

Par exemple, dans le brevet américain US 4 481 076, il est proposé de sécher des microfibrilles de cellulose issues de pâte de bois en présence d'additif. Les teneurs les plus favorables à une bonne redispersion après séchage, et donc à un bon niveau de viscosité de la suspension, sont de l'ordre de 50 à 100 % en poids par rapport aux microfibrilles sèches. Comme on peut le constater, les quantités d'additifs introduites sont très importantes. Par ailleurs, ces méthodes ne donnent pas entièrement satisfaction, même s'il est a priori possible de redisperser ces microfibrilles séchées. Les moyens mis en oeuvre pour la redispersion sont en effet toujours très cisaillants.

Dans la demande internationale WO 95102966, il est décrit l'additivation de cellulose microcristalline avec de la gomme xanthane ou de la carboxyméthylcellulose, avec des teneurs inférieures à 33% en poids par rapport au poids de cellulose microcristalline. Cependant, les conditions de mise en suspension de la cellulose séchée sont très fortement cisaillantes, car elles sont faites dans les conditions classiques d'agitation de formulations destinées à des applications dans le domaine de l'alimentaire. Les microfibrilles séchées ne peuvent donc pas être considérées comme facilement redispersables.

L'enseignement apporté par l'art antérieur sur la redispersion des microfibrilles de cellulose microcristalline, et notamment celles issues de la pâte de bois, ne peut pas être transposé aux nanofibrilles de cellulose, issues de cellules à parois primaires.

Tout d'abord, les microfibrilles de cellulose issues du bois, proviennent de parois secondaires. Cela signifie qu'elles ont un taux de cristallinité supérieur à 70 %. Lors de l'étape d'homogénéisation des microfibrilles issues du bois, on constate, non pas un désenchevêtrement des fibres comme c'est le cas lors de l'étape d'homogénéisation des nanofibrilles de cellulose issues de parois primaires, mais une cassure de ces fibrilles. Par conséquent, les microfibrilles de cellulose issues de parois secondaires, n'ont pas les caractéristiques des fibrilles amorphes mais, au contraire ont les caractéristiques de microfibrilles microcristallines.

Par ailleurs, les morphologies des microfibrilles et des nanofibrilles sont différentes. En effet, les microfibrilles microcristallines, par exemple issues de cellulose à parois secondaires, comme la pâte de bois, se présentent classiquement sous la forme d'agrégats de quelques dizaines de nanomètres à quelques micromètres, constitués de fibrilles élémentaires, qui ne peuvent pas être désenchevêtrées, lors de l'étape d'homogénéisation. En ce qui concerne les nanofibrilles de cellulose issues de cellules à parois primaires, elles présentent un diamètre d'au plus quelques nanomètres et ont l'aspect de filaments.

Il est relativement bien établi que la difficulté de redisperser des microfibrilles ou des nanofibrilles de cellulose est liée à l'existence de nombreuses liaisons hydrogène entre les fibrilles, créées lors du séchage. Or le nombre des liaisons hydrogène par unité de poids de cellulose, est directement relié à la morphologie desdites microfibrilles ou nanofibrilles, et plus précisément, est proportionnel à leur surface spécifique ; plus elle est élevée et plus le nombre de liaisons hydrogène par unité de poids de cellulose est important. Étant donné la morphologie particulière des nanofibrilles de cellulose issues de cellules à parois primaires, la surface spécifique de ces dernières est beaucoup plus élevée que celle des microfibrilles. L'homme de l'art se serait donc attendu, logiquement, à rencontrer des difficultés accrues pour redisperser des nanofibrilles de cellulose.

Ainsi, étant donné l'état de la technique présenté ci-dessus, il était prévisible que des quantités d'additif plus élevées que celles mises en oeuvre pour les microfibrilles seraient nécessaires pour obtenir une bonne redispersion des nanofibrilles séchées.

Or, la présente invention a montré, contre toute attente, que des quantités d'additif relativement faibles étaient suffisantes pour permettre une bonne redispersion des nanofibrilles séchées, et cela sans qu'il soit nécessaire de mettre en oeuvre des conditions très fortement cisaillantes. En outre, il a été trouvé de façon surprenante que des quantités de l'ordre de celles préconisées dans l'art antérieur, présentaient des inconvénients importants pour la conservation des propriétés rhéologiques des nanofibrilles.

Ceci provient de la différence de comportement entre les microfibrilles cristallines, par exemple les microfibrilles de cellulose issues de parois secondaires, et les nanofibrilles issues de cellules à parois primaires.

En effet, les microfibrilles microcristallines non additivées, ne sont pas dispersables en milieu aqueux ; elles décantent immédiatement après arrêt de l'agitation, même en mettant en oeuvre des moyens d'agitation très cisaillants. De plus, elles ne confèrent pas de propriétés rhéologiques rhéofluidifiantes.

Par contre, les nanofibrilles issues de parois primaires présentent un caractère dispersable en milieux aqueux. Elles apportent, en outre, un profil rhéologique bien spécifique, de type rhéofluidifiant, au milieu dans lequel elle sont introduites.

Or d'une manière générale, le séchage altère non seulement la capacité à la redispersion des nanofibrilles séchées et leur viscosité mais également, leur profil rhéologique. Ainsi, de grandes quantités d'additifs du type de celles mises en oeuvre habituellement pour redisperser des microfibrilles microcristallines, comme celles issues du bois, c'est-à-dire autant d'additif que de microfibrilles, ne donnent pas de bons résultats en ce qui concerne le profil rhéologique rhéofluidifiant des nanofibrilles de cellulose issues de parois primaires : le profil devient plus newtonien, c'est-à-dire moins rhéofluidifiant.

Comme on peut le constater, les conséquences du séchage des nanofibrilles de cellulose essentiellement amorphes sur la redispersion de ces fibrilles ainsi que leurs propriétés rhéologiques (viscosité à faible et fort cisaillement, profil rhéologique) ne peut être résolu de manière satisfaisante en se basant sur les connaissances apportées par l'additivation de microfibrilles microcristallines, par exemple de microfibrilles issues de cellules à parois secondaires.

La présente invention apporte donc une solution simple et efficace à ces problèmes.

Ces buts et d'autres sont atteints par la présente invention qui a pour premier objet une composition comprenant des nanofibrilles de cellulose essentiellement amorphes, au moins un additif choisi parmi la cellulose carboxylée présentant un degré de substitution supérieur à 0,95, un polysaccharide naturel, un polyol, et éventuellement au moins un co-additif, la teneur en additif et en co-additif éventuel étant inférieure ou égale à 30 % en poids par rapport au poids de nanofibrilles et d'additif et de co-additif éventuel.

Un autre objet de la présente invention est constitué par un procédé de préparation d'une composition, dans lequel on prépare des nanofibrilles de cellulose à partir de pulpe cellulosique en effectuant au moins une extraction puis éventuellement au moins une étape de blanchiment de la pulpe ainsi traitée, puis on sépare la pulpe résultante, et l'on met en oeuvre une étape d'homogénéisation en au moins un cycle, la caractéristique du procédé étant que l'on effectue les étapes suivantes :
- on ajoute à la suspension de nanofibrilles, éventuellement ayant subi au moins un cycle d'homogénéisation, au moins une partie de l'additif et éventuellement du ou des co-additifs,
- on effectue une étape de séchage de la suspension ainsi additivée.

Un troisième objet de l'invention a trait à une suspension comprenant des nanofibrilles de cellulose, obtenue en redispersant la composition selon l'invention.

La présente invention permet à la fois de proposer un procédé de séchage de nanofibrilles essentiellement amorphes en présence d'additifs, ainsi que des compositions séchées telles qu'elles soient facilement redispersables, tout en conservant les propriétés rhéologiques spécifiques des suspensions initiales, non séchées. Ainsi, les suspensions selon l'invention, obtenues après redispersion des compositions présentent un bon niveau de viscosité à faible gradient de cisaillement, ainsi qu'un profil rhéologique de type rhéofluidifiant.

En outre, les moyens mis en oeuvre pour redisperser les compositions séchées selon l'invention, sont considérablement moins cisaillants que ceux habituellement employés pour redisperser des microfibrilles séchées issues de bois, ou d'autres parois secondaires.

D'autres caractéristiques et avantages de la présente invention apparaîtront plus clairement à la lecture de la description et des exemples qui vont suivre.

Ainsi que cela a été indiqué auparavant, la présente invention a pour objet l'additivation de nanofibrilles de cellulose, essentiellement amorphes.

Par essentiellement amorphes, on entend des nanofibrilles dont le taux de cristallinité est inférieur ou égal à 50 %. Selon une variante particulière de la présente invention, le taux de cristallinité est compris entre 15 % et 50 %. Dé préfeérnce, le taux de cristallinité est inférieur à 50 %.

Les nanofibrilles de cellulose traitées selon la présente invention sont issues de cellules constituées, de préférence, d'au moins environ 80% de parois primaires. De préférence, la quantité de parois primaires est d'au moins 85 % en poids.

On a de telles caractéristiques notamment avec des cellules de parenchyme. La pulpe de betterave sucrière, les citrus comme les citrons, les oranges, les pamplemousses, et la plupart des fruits et des légumes constituent des exemples de parenchyme.

Par ailleurs, les nanofibrilles entrant dans les compositions selon l'invention sont, selon une variante particulièrement avantageuse, chargées en surface en acides carboxyliques et en polysaccharides acides, seuls ou en mélange.

Par acides carboxyliques, on entend les acides carboxyliques simples, ainsi que leurs sels. Ces acides sont de préférence choisis parmi les acides uroniques. Plus particulièrement, lesdits acides uroniques sont plus particulièrement l'acide galacturonique, l'acide glucuronique.

En tant que polysaccharides acides, on peut citer les pectines, qui sont plus particulièrement des acides polygalacturoniques. Ces polysaccharides acides peuvent être présents en mélange avec des hémicelluloses.

Les nanofibrilles de cellulose présentent en outre une section comprise entre environ 2 et environ 10 nm. Plus particulièrement, la section des nanofibrilles est comprise entre environ 2 et environ 4 nm.

Selon un mode de réalisation particulièrement avantageux de la présente invention, les nanofibrilles entrant dans les compositions selon l'invention sont obtenues en mettant en oeuvre le traitement qui va être décrit ci-dessous. Plus particulièrement, ce traitement est effectué sur de la pulpe de végétaux à parois primaires, comme par exemple de la pulpe de betterave après que celle-ci a subi une étape d'extraction préalable du saccharose, selon les méthodes connues de la technique.

Ainsi, le procédé comprend les étapes suivantes :
(a) première extraction acide ou basique, à l'issue de laquelle on récupère un premier résidu solide,
(b) éventuellement seconde extraction effectuée dans des conditions alcalines du premier résidu solide, à la suite de quoi, est récupéré un second résidu solide,
(c) lavage du premier ou du second résidu solide,
(d) éventuellement blanchiment du résidu lavé,
(e) dilution du troisième résidu solide obtenu à l'issue de l'étape (d) de manière à obtenir un taux de matières sèches compris entre 2 et 10 % en poids,
(f) homogénéisation de la suspension diluée.

Dans l'étape (a), on entend par "pulpe" de la pulpe humide, déshydratée, conservée par ensilage ou partiellement dépectinée.

L'étape d'extraction (a) peut être effectuée en milieu acide ou en milieu basique.

Pour une extraction acide, la pulpe est mise en suspension dans une solution d'eau pendant quelques minutes de façon à homogénéiser la suspension acidifiée à un pH compris entre 1 et 3, de préférence entre 1,5 et 2,5.

Cette opération est mise en oeuvre avec une solution concentrée d'un acide tel que l'acide chlorhydrique ou l'acide sulfurique.

Cette étape peut être avantageuse pour éliminer les cristaux d'oxalate de calcium qui peuvent être présents dans la pulpe, et qui, du fait de leur caractère abrasif important, peuvent causer des difficultés dans l'étape d'homogénéisation.

Pour une extraction basique la pulpe est ajoutée à une solution alcaline d'une base, par exemple de la soude ou de la potasse, de concentration inférieure à 9 % en poids, plus particulièrement inférieure à 6 % en poids. De préférence, la concentration de la base est comprise entre 1 et 2 % en poids.

On pourra ajouter une faible quantité d'un agent antioxydant soluble dans l'eau, tel que le sulfite de sodium Na₂SO₃, afin de limiter les réactions d'oxydation de la cellulose.

L'étape (a) est effectuée en général à une température comprise entre environ 60°C et 100°C, de préférence comprise entre environ 70°C et 95°C.

La durée de l'étape (a) est comprise entre environ 1 heure et environ 4 heures.

Lors de l'étape (a), il se produit une hydrolyse partielle avec libération et solubilisation de la majeure partie des pectines et des hémicelluloses, tout en préservant la masse moléculaire de la cellulose.

Le résidu solide est récupéré à partir de la suspension provenant de l'étape (a) en mettant en oeuvre des méthodes connues. Ainsi, il est possible de séparer le résidu solide par centrifugation, par filtration sous vide ou sous pression, avec les toiles filtrantes, ou les filtres-presses par exemple, ou encore par évaporation.

On soumet éventuellement le premier résidu solide obtenu à une seconde étape d'extraction, effectuée dans des conditions alcalines.

On met en oeuvre une seconde étape d'extraction, étape (b), lorsque la première a été conduite dans des conditions acides. Si la première extraction a été effectuée dans des conditions alcalines, la seconde étape n'est que facultative.

Selon le procédé, cette seconde extraction est effectuée avec une base, de préférence choisie parmi la soude et la potasse, dont la concentration est inférieure à environ 9 % en poids, de préférence comprise entre environ 1 % et environ 6 % en poids.

La durée de l'étape d'extraction alcaline est comprise entre environ 1 et environ 4 heures. Elle est de préférence égale à environ 2 heures.

A l'issue de cette seconde extraction, si elle a lieu, on récupère un second résidu solide.

Dans l'étape (c) le résidu provenant de l'étape (a) ou (b) est lavé abondamment à l'eau afin de récupérer le résidu de matériau cellulosique.

Le matériau cellulosique de l'étape (c) est ensuite facultativement blanchi, dans l'étape (d), selon les méthodes classiques. Par exemple, on peut effectuer un traitement au chlorite de sodium, à l'hypochlorite de sodium, au peroxyde d'hydrogène à raison de 5-20 % par rapport à la quantité de matières sèches traitée.

Différentes concentrations d'agent de blanchiment peuvent être utilisées, à des températures comprises entre environ 18°C et 80°C, de préférence entre environ 50°C et 70°C.

La durée de cette étape (d) est comprise entre environ 1 heure et environ 4 heures, de préférence entre environ 1 et environ 2 heures.

On obtient alors un matériau cellulosique contenant entre 85 et 95 % en poids de cellulose.

A l'issue de cette étape de blanchiment, il peut être préférable de laver abondamment la cellulose avec de l'eau.

La suspension résultante, éventuellement blanchie, est ensuite rediluée dans de l'eau à raison de 2 à 10 % de matières sèches (étape (e)), avant de subir une étape d'homogénéisation (étape (f)), comprenant au moins un cycle.

Selon une première variante de l'invention, les nanofibrilles sont additivées avant de subir l'étape d'homogénéisation.

Selon une seconde variante de l'invention, les nanofibrilles de cellulose sont additivées après avoir subi au moins un cycle d'homogénéisation.

L'étape d'homogénéisation correspond à un mixage, broyage ou toute opération de cisaillement mécanique élevé, suivie d'un ou plusieurs passages de la suspension de cellules à travers un orifice de petit diamètre, soumettant la suspension à une chute de pression d'au moins 20 mPa et à une action de cisaillement à vitesse élevée suivie d'un impact de décélération à vitesse élevée.

Le mixage ou broyage est, par exemple, effectué par passage(s) au mixeur ou broyeur pendant une durée allant de quelques minutes à environ une heure, dans un appareil de type tel un WARING BLENDOR équipé d'une hélice à quatre pales ou broyeur à meule ou tout autre type de broyeur, tel un broyeur colloïdal.

L'homogénéisation proprement dite sera avantageusement effectuée dans un homogénéiseur du type MANTON GAULIN dans lequel la suspension est soumise à une action de cisaillement à vitesse et à pression élevées dans un passage étroit et contre un anneau de choc. On peut aussi citer le MICRO FLUIDIZER qui est un homogénéiseur principalement constitué d'un moteur à air comprimé qui va créer de très fortes pressions, d'une chambre d'interaction dans laquelle s'effectuera l'opération d'homogénéisation (cisaillement élongationnel, chocs et cavitations) et d'une chambre basse pression qui permet la dépressurisation de la dispersion.

La suspension est introduite dans l'homogénéisateur de préférence après préchauffage à une température comprise entre 40 et 120°C, de préférence comprise entre 85 et 95°C.

La température de l'opération d'homogénéisation est maintenue entre 95 et 120°C, de préférence supérieure à 100°C.

La suspension est soumise dans l'homogénéisateur à des pressions comprises entre 20 et 100 mPa, et de préférence supérieures à 50 mPa.

L'homogénéisation de la suspension cellulosique est obtenue par un nombre de passages pouvant varier entre 1 et 20, de préférence entre 2 et 5, jusqu'à l'obtention d'une suspension stable.

L'opération d'homogénéisation peut avantageusement être suivie d'une opération de cisaillement mécanique élevé, par exemple dans un appareil tel l'ULTRA TURRAX de SYLVERSON.

Il est à noter que ce procédé a été décrit dans la demande de brevet européen EP 726 356 déposée le 07/02/96, on pourra donc s'y référer si nécessaire. L'exemple 20 de ce texte donne notamment un mode de préparation de suspension de nanofibrilles de cellulose essentiellement amorphes.

Les additifs vont maintenant être décrits.

Le premier additif, ou simplement l'additif, entrant dans la composition selon l'invention est choisi parmi la cellulose carboxylée, sous forme sel ou acide, présentant un degré de substitution supérieur à 0,95, un polysaccharide naturel, un polyol.

Les additifs mentionnés peuvent être présents seuls ou en mélanges.

Selon un premier mode de réalisation, l'additif de la composition selon l'invention est constitué par de la cellulose carboxylée présentant un degré de substitution particulier.

La cellulose employée comme additif est, plus particulièrement, de la cellulose carboxyméthylée. La cellulose est un polymère constitué d'unités monomériques de glucose. Le groupement carboxylé est introduit de manière connue en soi, en faisant réagir l'acide chloro-acétique avec la cellulose.

Le degré de substitution correspond au nombre de groupements carboxyméthylés par unité de glucose. Le degré théorique maximal est de 3.

Selon l'invention, le degré de substitution de la cellulose carboxyméthylée est donc supérieur à 0,95.

Le degré de polymérisation de la cellulose carboxylée employée comme additif des nanofibrilles, conformément à la présente invention, varie dans de larges limites. Ainsi, conviennent les celluloses carboxyméthylées de fortes masses (degré de polymérisation élevé, viscosité élevée) ou de faibles masses (degré de polymérisation faible, viscosité faible).

Dans la première catégorie, on peut mentionner les celluloses dont la viscosité est comprise entre environ 9000 mPa.s mesurée dans une solution dans l'eau à 1 % (Brookfield, 30 tr/mn) et 250 mPa.s mesurée dans une solution dans l'eau à 6 % (Brookfield 60 tr/mn).

Dans la seconde catégorie, on peut mentionner les celluloses dont la viscosité est comprise entre environ 250 mPa.s mesurée dans une solution dans l'eau à 6 % (Brookfield, 60 tr/mn) et 10 mPa.s mesurée dans une solution dans l'eau à 6 % (Brookfield, 60 tr/mn).

Dans le cas de la première catégorie, la teneur en cellulose carboxylée est inférieure ou égale à 30 % en poids.

Dans le cas de la seconde catégorie, la teneur en cellulose carboxylée est plus particulièrement comprise entre 10 et 30 % en poids.

L'additif entrant dans la composition selon l'invention peut aussi être un polysaccharide naturel.

Ainsi, le polysaccharide peut être d'origine bactérienne, animale ou végétale.

Les polysaccharides sont des polymères comprenant des unités osidiques. De préférence, on met en oeuvre des polysaccharides se trouvant sous une forme anionique ou non ionique.

Parmi les polysaccharides anioniques convenables, on peut mentionner sans intention de s'y limiter, la gomme xanthane, les succinoglycanes, les carraghénanes, les alginates.

A titre de polysaccharides non ioniques, on peut citer par exemple les galactomannanes comme la gomme de guar, la gomme de caroube. Convient aussi l'amidon et ses dérivés non ioniques, de même que les dérivés non ioniques de la cellulose.

Selon une variante particulière de mise en oeuvre de l'invention, on utilise comme additif un polysaccharide anionique et plus spécialement la gomme xanthane.

Parmi les polyols convenables, on peut citer tout particulièrement l'alcool polyvinylique.

Un mode de réalisation préféré de la présente invention est constitué par une composition dont l'additif est la cellulose carboxylée telle qu'elle a été définie.

Un second mode de réalisation particulier est constitué par un additif comprenant un polysaccharide, de préférence anionique, éventuellement combiné à la cellulose carboxylée précitée.

La composition selon l'invention peut comprendre, en outre, au moins un co-additif choisi parmi :
- la cellulose carboxylée présentant un degré de substitution inférieur ou égal à 0,95, de préférence de la cellulose carboxyméthylée,
- les monomères ou oligomères osidiques,
- les composés de formule (R¹R²N)COA, formule dans laquelle R¹ ou R², identiques ou différents, représentent l'hydrogène ou un radical alkyle en C₁-C₁₀, de préférence en C₁-C₅, A représente l'hydrogène, un radical alkyle en C₁-C₁₀, de préférence en C₁-C₅, ou encore le groupement R'¹R'²N avec R'¹, R'², identiques ou différents, représentant l'hydrogène ou un radical alkyle en C₁-C₁₀, de préférence en C₁-C₅,
- les tensioactifs cationiques ou amphotères,
ces co-additifs pouvant être utilisés seuls ou en mélange.

Il est à noter que ce qui a été indiqué sur la nature et les viscosités de la cellulose carboxylée, et plus particulièrement la cellulose carboxyméthylée présentant un haut degré de substitution reste valable ici, à l'exception du degré de substitution.

Parmi les monomères ou oligomères osidiques, on peut citer tout particulièrement et sans intention de se limiter, le sorbitol, le saccharose, le fructose.

En ce qui concerne les composés du type (R¹R²N)COA, on préfère utiliser les composés comprenant deux fonctions amides. De préférence on utilise l'urée comme co-additif.

Parmi les tensioactifs cationiques, on peut citer les dérivés cationiques d'ammonium quaternaires, comme par exemple les dérivés cationiques d'imidazoline, les halogénures d'alkyltriméthylammonium, de dialkyldiméthylammonium, d'alkyldiméthyl-benzylammonium, d'alkyldiméthyléthylammonium, les Esters Quat.

A titre d'exemple de composés cationiques convenables, on peut citer les produits commercialisés par Rhône-Poulenc de la gamme Rhodaquat. On peut aussi utiliser des polymères cationiques synthétiques, connus sous le nom générique CTFA de "Polyquaternium", par exemple les polymères MIRAPOL A15® ou MIRAPOL 550® de la société Rhône-Poulenc.

Les tensioactifs entrant dans la formulation selon l'invention peuvent aussi être choisis parmi les tensioactifs amphotères. A titre d'exemple, on peut citer sans intention de se limiter, les dérivés amphotères d'alkylpolyamines, les alkylbétaines, les alkyldiméthylbétaines, les alkylamidopropylbétaines, les alkylamidopropyldiméthylbétaines, les alkyltriméthyl-sulfobétaines, les dérivés d'imidazoline tels que les alkylamphoacétates, alkylamphodiacétates, alkylamphopropionates, alkylamphodipropionates, les alkylsultaines ou les alkylamidopropyl-hydroxysultaines, les produits de condensation d'acides gras et d'hydrolysats de protéines, ces composés pouvant être utilisés seuls ou en mélange.

Les tensioactifs Mirapon® Excel, Mirataine® CBS, Mirataine® CB, Mirataine H2C-HA®, Ampholac 7T/X®, Ampholac 7C/X, la gamme des Miranol®, Amphionic® SFB, Amphionic® XL peuvent notamment convenir à la réalisation de la présente invention.

Lorsque les compositions selon l'invention comprennent un ou plusieurs des co-additifs précités, leur teneur est inférieure à 30 % en poids par rapport au poids de nanofibrilles et d'additif et de co-additif. Bien entendu, la teneur en additif(s) et en co-additif(s) est telle qu'elle est inférieure ou égale à 30 % par rapport au poids de nanofibrilles, d'additif(s) et de co-additif(s).

Selon une première variante particulière de l'invention, les compositions comprennent au moins un additif, ainsi qu'au moins un co-additif choisi parmi la cellulose carboxylée présentant un degré de substitution inférieur ou égal à 0.95, les monomères ou oligomères osidiques ou les composés de formule (R¹R²N)COA.

Dans le cas de cette première variante, la teneur en co-additif est inférieure à 30 % et de préférence comprise entre 1 et 25 % en poids par rapport au poids de nanofibrilles et d'additif et de co-additif.

Selon une seconde variante particulière de l'invention, les compositions comprennent au moins un additif et, en tant que co-additif. au moins un composé choisi parmi les tensioactifs cationiques ou amphotères.

Dans le cas de cette seconde variante, la teneur en co-additif est comprise entre 1 et 10 % en poids par rapport au poids de nanofibrilles et d'additif et de co-additif.

Dans chacune des deux variantes, la teneur en additif est inférieure ou égale à 30 % en poids, par rapport au poids de nanofibrilles et d'additif et de co-additif.

Dans le cas d'additifs de redispersion tels que la cellulose carboxylée de haut degré de substitution (degré de substitution supérieur à 0,95) ou de co-additifs comme la cellulose à bas degré de substitution (degré de substitution inférieur ou égal à 0,95), plus sa concentration est élevée et plus elle diminue le caractère rhéofluidifiant des nanofibrilles de cellulose en modifiant leur état de dispersion dans l'eau. Ainsi, dans le cas de la cellulose carboxylée et pour des concentrations supérieures à 30 % en poids par rapport au poids de nanofibrilles et d'additif et de co-additif, bien que les nanofibrilles soient redispersables, leur profil rhéologique devient plus newtonien, c'est-à-dire moins rhéofluidifiant, ce qui n'est pas souhaitable.

Dans le cas de l'emploi de cellulose carboxylée présentant un haut degré de substitution, en comparaison à une cellulose carboxylée à faible degré de substitution, on a constaté que la cellulose carboxylée de haut degré de substitution s'avérait plus efficace en pouvoir redispersant et en maintien du profil rhéologique rhéofluidifiant des nanofibrilles de cellulose. Ainsi, à masse de cellulose carboxylée analogue, la concentration en cellulose carboxylée de haut degré de substitution nécessaire peut être avantageusement réduite par rapport à une cellulose carboxylée de bas degré de substitution.

Ainsi, ladite teneur en additif et en co-additif éventuel peut être choisie inférieure ou égale à 25% en poids par rapport au poids de nanofibrilles et d'additif et de co-additif éventuel; cette teneur est de préférence comprise entre 5 % et 25 % en poids par rapport à la même référence; ainsi les nanofibrilles de cellulose se redispersent aisément et conservent leurs propriétés rhéologiques rhéofluidifiants.

Notons que l'utilisation de tels co-additifs décrits précédemment permet, en combinaison avec la carboxyméthylcellulose, de renforcer le profil rhéofluidifiant des nanofibrilles de cellulose après redispersion.

En outre les compositions selon l'invention, présentent une teneur en matières sèches d'au moins 40 % en poids. Plus particulièrement, la teneur en matières sèches est d'au moins 60 %, de préférence, elle est d'au moins 70 % en poids.

D'une manière avantageuse, on a remarqué que le profil rhéologique de suspensions séchées jusqu'à obtenir un taux de matières sèches de cet ordre, n'est pas altéré.

La granulométrie de la composition selon l'invention peut varier dans de larges limites. Elle est habituellement comprise entre 1 µm à quelques millimètres.

Le procédé de préparation des compositions va maintenant être décrit plus en détails.

Le procédé selon l'invention consiste, tout d'abord, à préparer les nanofibrilles de cellulose à partir de pulpe cellulosique appropriée en effectuant une hydrolyse puis éventuellement au moins une étape de blanchiment de la pulpe ainsi traitée. Ce qui a été indiqué auparavant à ce propos reste valable et ne sera pas repris ici.

Le procédé de préparation des compositions selon l'invention consiste, dans une première étape, à ajouter à la suspension de nanofibrilles, éventuellement ayant subi au moins un cycle d'homogénéisation, au moins une partie de l'additif et éventuellement du ou des co-additifs. Puis, dans une seconde étape, on met en oeuvre une étape de séchage de la suspension ainsi additivée.

Selon une première variante avantageuse de la présente invention, l'addition d'au moins une partie de l'additif et éventuellement du ou des co-additifs est effectuée à l'issue de l'étape d'homogénéisation.

Un mode de réalisation particulièrement approprié de l'invention consiste à ajouter au moins une partie de l'additif et éventuellement du ou des co-additifs, à la suspension à l'issue de l'étape d'homogénéisation, après que cette dernière a subi au moins une étape de concentration.

L'étape ou les étapes de concentration ont lieu par filtration, centrifugation, ou encore évaporation d'une partie de l'eau de la suspension. On peut, par exemple, utiliser des filtres sous vide ou sous pression, des tours d'atomisation, des fours, des fours à micro-ondes.

On peut aussi effectuer une précipitation, par exemple dans un alcool tel que réthanol, l'isopropanol ou tout autre alcool semblable, mettre en oeuvre un procédé de séparation par congélation-décongélation, par dialyse contre une solution hygroscopique dont la taille des molécules est supérieure à la taille des pores de la membrane utilisée.

Ces méthodes ne sont citées qu'à titre indicatif et ne peuvent être considérées comme une liste exhaustive.

Selon ce mode de réalisation, l'opération de concentration peut être conduite jusqu'à obtenir un extrait sec d'environ 35 % en poids. Plus particulièrement, l'extrait sec est compris entre 5 et 25 % en poids.

L'introduction de l'additif et éventuellement du ou des co-additifs est effectuée de manière connue en soi, c'est-à-dire par tout moyen permettant d'introduire de manière homogène une solution, une suspension ou une poudre, à une suspension qui a plutôt la consistance d'une pâte. Par exemple, on peut citer les broyeurs, les extrudeurs, les malaxeurs.

Cette opération peut être effectuée dans une large gamme de température, comprise plus particulièrement entre la température ambiante et 80°C. Il peut être avantageux d'effectuer l'introduction à la température à laquelle a eu lieu la concentration. Il est à noter en outre que des températures de l'ordre de 50 à 80°C peuvent aussi faciliter l'ajout de l'additif, en diminuant par exemple sa viscosité.

Un second mode de réalisation du procédé consiste à ajouter au moins une partie de l'additif et éventuellement du ou des co-additifs, à la suspension à l'issue de l'étape d'homogénéisation, avant que cette dernière ait subi au moins une étape de concentration.

Dans ce dernier cas, l'étape ou les étapes de concentration qui ont lieu après l'ajout d'additif et éventuellement de co-additif, sont effectuées de la même manière que précédemment indiqué.

Un mode de réalisation préféré de l'invention, si cette première variante est mise en oeuvre, est d'effectuer l'additivation après que la suspension a subi une ou plusieurs étapes de concentration.

Selon une deuxième variante avantageuse de la présente invention, l'addition d'au moins une partie de l'additif et éventuellement du ou des co-additifs est effectuée avant ou pendant l'étape d'homogénéisation. Lorsqu'il est indiqué que l'additivation a lieu pendant l'étape d'homogénéisation, on entend que l'additif et éventuellement le(s) co-additif(s) sont introduits alors que la pulpe a subi au moins un cycle de l'étape d'homogénéisation.

L'additivation a lieu selon les méthodes indiquées dans le cadre de la première variante.

Préalablement à l'étape de séchage proprement dite, il peut être avantageux d'effectuer une mise en forme de la suspension qui a été concentrée comme mentionné auparavant.

Cette mise en forme est réalisée de manière connue de l'homme du métier. On peut notamment citer, sans intention de s'y limiter toutefois, l'extrusion, la granulation.

La première est effectuée dans les appareillages classiques comprenant tout type de filière, la seconde peut être effectué, par exemple, dans des tambours, drageoirs.

Le séchage est réalisé par tout moyen connu de l'homme du métier, dans la mesure où ce moyen permet d'avoir une bonne homogénéité de la température de la suspension, mise en forme ou non.

A ce titre, on peut citer l'évaporation dans des fours sur tapis roulant, à induction ou non, radiatifs ou non, des fours rotatifs ou encore les lits fluidisés, ou dans un lyophilisateur.

Selon une variante particulièrement avantageuse de la présente invention, on effectue l'étape de séchage de manière à maintenir au minimum 3 % en poids d'eau par rapport au poids du solide obtenu. Plus particulièrement, le poids d'eau maintenu est compris entre 10 et 30% en poids. Une telle mise en oeuvre permet de ne pas dépasser le seuil au-delà duquel la redispersion des nanofibrilles ne peut plus être complète.

Le séchage a lieu de manière avantageuse sous air, bien qu'il soit envisageable de le mettre en oeuvre sous un gaz inerte, comme l'azote.

Il est en outre à noter que l'on préfère mettre en oeuvre le séchage sous une atmosphère dont le degré d'humidité est contrôlé de manière à pouvoir maintenir le taux d'humidité souhaité dans la composition.

La température de séchage doit limiter toute dégradation des acides carboxyliques, des polysaccharides acides, des hémicelluloses et/ou des additifs et co-additifs. Elle est plus particulièrement comprise entre 30 et 80°C, de préférence entre 30 et 60°C.

Il est à noter que l'on ne sortirait pas du cadre de la présente invention en mettant en oeuvre un séchage en plusieurs étapes, dont certaines d'entre elles mettraient en oeuvre les moyens indiqués précédemment pour l'étape de concentration.

A l'issue de l'étape de séchage, on peut effectuer un broyage de la composition obtenue.

Si une telle possibilité est retenue, la granulométrie de la poudre est en général comprise entre 1 µm et quelques millimètres, de préférence entre 30 et quelques millimètres. Une telle granulométrie permet de faciliter dans une certaine mesure la redispersion, tout en limitant les problèmes de manipulation.

Un autre objet de la présente invention est constitué par une suspension de nanofibrilles de cellulose obtenue par redispersion dans l'eau ou tout autre milieu, de la composition additivée selon l'invention.

La suspension selon l'invention, outre le fait qu'elle est susceptible d'être obtenue par redispersion de la composition selon l'invention, présente un profil rhéologique de type rhéofluidifiant.

Par ailleurs, elle présente un niveau de viscosité correspondant à au moins 50 % pour un taux de cisaillement d'au moins 1 s⁻¹, du niveau de viscosité d'une suspension de nanofibrilles de cellulose n'ayant pas subi d'étape de séchage et ne comprenant pas d'additifs, ni de co-additifs.

La présente invention a aussi pour objet l'utilisation de cellulose carboxylée, de préférence, de la carboxyméthylcellulose, et éventuellement de co-additifs, avec des nanofibrilles de cellulose essentiellement amorphes, dans le but de conserver un profil rhéologique rhéofluidifiant à une suspension comprenant des nanofibrilles de cellulose essentiellement amorphes ayant subi une étape de séchage.

Tout ce qui a été indiqué auparavant sur les additifs, co-additifs ainsi que les autres éléments constitutifs de la composition selon l'invention, de même que la préparation de ladite composition reste valable et l'on pourra s'y référer.

Les compositions selon l'invention, ainsi que les suspensions obtenues par redispersion des premières, peuvent être utilisées dans de nombreux domaines où l'on souhaite avoir un profil rhéologique rhéofluidifiant. Cela peut être le cas pour des fluides employés pour l'exploitation pétrolière, pour des formulations destinées au domaine de la cosmétique, de la détergence, alimentaires, ou encore des travaux publics et le bâtiment.

Des exemples concrets mais non limitatifs de l'invention vont maintenant être présentés.

### EXEMPLE 1 COMPARATIF :

L'exemple comparatif est effectué en l'absence d'additif ou de co-additif.

La dispersion-mère de nanofibrilles utilisée contient 2,3 % en poids de nanofibrilles de cellulose, fournie par la GENERALE SUCRIERE, et est préhomogénéisée à l'Ultra-Turrax à 14000 tr/mn (1 mn pour 100 g de dispersion).

Cette dispersion-mère non séchée, est ensuite diluée à 0,3 % en poids de nanofibrilles de cellulose dans de l'eau distillée à l'aide de l'Ultra-Turrax à 8000 tr/mn pendant 1 mn. Elle constitue la solution témoin.

La même dispersion-mère est concentrée jusqu'à un extrait sec de 40 % à l'aide d'un filtre-presse. Le solide obtenu est ensuite redispersé à 0,3 % en poids de nanofibrilles de cellulose dans de l'eau distillée. L'agitation s'effectue à l'Ultra-Turrax à 8000 tr/mn pendant 1 mn. Le mélange 1 est alors obtenu.

Une rhéologie en écoulement est effectuée au bout de 24 heures sur un rhéomètre RFS 8400 en géométrie Couette (balayage en gradient de cisaillement entre 1 et 100 s⁻¹).

Les résultats sont récapitulés dans le tableau 1.

**Tableau 1**

| **gradient de cisaillement** **(s**^{**-1**}**)** | **viscosité (Pa.s)** | **viscosité (Pa.s)** |
|---|---|---|
| | **témoin** | **mélange 1** |
| 1,27 | 3,0 | 2,0.10⁻¹ |
| 2,01 | 1,3 | 9,6.10⁻² |
| 5,05 | 4,3.10⁻¹ | 4,2.10⁻² |
| 12,7 | 1,6.10⁻¹ | 2,3.10⁻² |
| 20,1 | 9,9.10⁻² | 1,8.10⁻² |
| 50,5 | 3,2.10⁻² | 8,8.10⁻³ |
| 80,0 | 1,6.10⁻² | 6,4.10⁻³ |

On constate que, dans le mélange 1, le volume de décantation (le surnageant) atteint 10 % après 4 heures de repos et dépasse 15 % au bout de 24 heures de repos, alors que le témoin reste stable.

De plus, la viscosité récupérée, après concentration sans additif et redispersion, n'est que de 7 % de la viscosité initiale pour un gradient de cisaillement supérieur ou égal à 1 s⁻¹.

L'exemple comparatif montre qu'en l'absence d'additif tel que le carboxyméthylcellulose de haut degré de substitution, le séchage des microfibrilles de cellulose suivi de la redispersion avec un outil très cisaillant (Ultra-Turrax) conduit à une dispersion instable qui a perdu 93 % de sa viscosité initiale pour un gradient de cisaillement supérieur ou égal à 1 s⁻¹.

### EXEMPLE 2 COMPARATIF

Cet exemple a pour objectif de montrer le comportement différent de microfibrilles de cellulose microcristalline.

### 1) Préparation des systèmes à base de microfibrilles de cellulose et de carboxyméthylcellulose de haut degré de substitution :

La carboxyméthylcellulose Blanose 12M8P® est mise en solution dans de l'eau distillée.

La solution est ensuite ajoutée à une suspension de microfibrilles de cellulose Acticel 12® (Active Organics) et l'ensemble est agité à l'Uttra-Turrax à 14000 tr/mn pendant 5 mn.

La quantité de carboxyméthylcellulose ajoutée est de 15 % en poids par rapport au poids de microfibrilles de cellulose et de carboxyméthylcellulose.

Le mélange est ensuite versé dans des coupelles puis séché dans un four à l'extrait sec de 97 %, contrôlé par dosage d'eau par la méthode KARL-FISCHER.

Le mélange séché est ensuite broyé au moulin à café, puis tamisé sur un tamis de 500 µm.

### 2) Redispersion des systèmes à base de microfibrilles de cellulose et de carboxyméthylcellulose de haut degré de substitution, et caractérisation :

La poudre obtenue est redispersée à 0,3 % en poids de microfibrilles de cellulose dans de l'eau distillée.
(a) L'agitation s'effectue à la pale défloculeuse à 1000 tr/mn pendant 30 mn.
   5 minutes après l'arrêt de l'agitation, une décantation a lieu dans laquelle le surnageant représente 91 % du volume.
(b) L'agitation s'effectue à l'Ultra-Turrax à 14000 tr/mn pendant 5 mn.
   5 minutes après l'arrêt de l'agitation, une décantation a lieu dans laquelle le surnageant représente 91 % du volume.

Cet exemple montre qu'il n'y a pas de redispersion des microfibrilles même lorsqu'elles sont soumises des conditions de très fort cisaillement. Par conséquent, des teneurs aussi faibles que 15 % d'additif par rapport aux microfibrilles microcristallin ne peuvent pas être mises en oeuvre pour redisperser des microfibrilles après séchage.

### EXEMPLE 3 COMPARATIF

Cet exemple a pour objectif de montrer le comportement différent de microfibrilles de cellulose microcristalline.

### 1) Préparation des systèmes à base de microfibrilles de cellulose et de gomme xanthane :

On reproduit l'exemple 2 comparatif, à l'exception du fait que l'additif est la gomme xanthane (Rhodopol 23®) et la quantité est de 30 % en poids par rapport au poids de microfibrilles de cellulose et de gomme xanthane.

### 2) Redispersion des systèmes à base de microfibrilles de cellulose et de gomme xanthane, et caractérisation :

La poudre obtenue est redispersée à 0,3 % en poids de microfibrilles de cellulose dans de l'eau distillée.
(a) L'agitation s'effectue à la pale défloculeuse à 1000 tr/mn pendant 30 mn.
   5 minutes après l'arrêt de l'agitation, une décantation a lieu dans laquelle le surnageant représente 90 % du volume.
(b) L'agitation s'effectue à l'Ultra-Turrax à 14000 tr/mn pendant 5 mn.
   5 minutes après l'arrêt de l'agitation, une décantation a lieu dans laquelle le surnageant représente 90 % du volume.

Cet exemple montre qu'il n'y a pas de redispersion des microfibrilles même lorsqu'elles sont soumises des conditions de très fort cisaillement. Par conséquent, des teneurs de l'ordre de 30 % d'additif par rapport aux microfibrilles microcristallines ne peuvent pas être mises en oeuvre pour redisperser des microfibrilles après séchage.

### EXEMPLE 4

### 1) Préparation des systèmes à base de nanofibrilles de cellulose et de carboxyméthylcellulose de haut degré de substitution :

La carboxyméthylcellulose (degré de substitution égal à 1,2; de viscosité moyenne - produit DRILLING SPECIALITIES COMPANY - DRISPAC SUPERLO) est mise en solution dans de l'eau distillée.

La solution est ensuite ajoutée à la dispersion-mère de nanofibrilles (2,9 % en nanofibrilles de cellulose fournie par la GENERALE SUCRIERE et préhomogénéisée à l'Ultra-Turrax à 14000 tr/mn (1 mn pour 100 g de dispersion)) et l'ensemble est agité à la pale défloculeuse à 1000 tr/mn pendant 30 mn.

La quantité de carboxyméthylcellulose ajoutée est de 15 à 30 % en poids, par rapport au poids de nanofibrilles de cellulose et de carboxyméthylcellulose.

Le mélange est ensuite versé dans des coupelles puis séché soit dans une étuve ventilée à 40°C, jusqu'à un extrait sec de 93 %, contrôlé par dosage d'eau par la méthode KARL-FISCHER.

Le mélange séché est ensuite broyé au moulin à café, puis tamisé sur un tamis de 500 µm.

### 2) Redispersion des systèmes à base de nanofibrilles de cellulose et de carboxyméthylcellulose de haut degré de substitution, et caractérisation :

La poudre obtenue est redispersée à 0,3 % en poids de nanofibrilles de cellulose dans de l'eau distillée. L'agitation s'effectue à la pale défloculeuse à 1000 tr/mn pendant 5 mn ou 30 mn.

Une rhéologie en écoulement est effectuée au bout de 24 heures sur un rhéomètre RFS 8400 en géométrie Couette (balayage en gradient de cisaillement entre 1 et 100 s⁻¹).

Tous les systèmes sont comparés aux nanofibrilles de cellulose non séchées et diluées dans l'eau à 0,3 % à l'Ultra-Turrax à 14000 tr/mn pendant 1 mn (état de redispersion optimal des nanofibrilles).

Le tableau II montre l'influence de la concentration en carboxyméthylcellulose (DRISPAC SUPERLO) sur le profil rhèologique des nanofibrilles de cellulose après redispersion.

**Tableau II**

| **gradient de cisaillement** **(s**^{**-1**}**)** | **viscosité (Pa.s)** | | |
|---|---|---|---|
| | **témoin** | **mélange 1** | **mélange 2** |
| 1,27 | 4,1.10⁻¹ | 5,6.10⁻¹ | 2,9.10⁻¹ |
| 2,01 | 2,6.10⁻¹ | 4,2.10⁻¹ | 1,9.10⁻¹ |
| 5,05 | 1,3.10⁻¹ | 2,5.10⁻¹ | 1,1.10⁻¹ |
| 12,7 | 1,0.10⁻¹ | 1,5.10⁻¹ | 7,3.10⁻² |
| 20,1 | 6,0.10⁻² | 1,2.10⁻¹ | 5,4.10⁻² |
| 50,5 | 2,8.10⁻² | 7,2.10⁻² | 3,5.10⁻² |
| 80,0 | 2,5.10⁻² | 5,7.10⁻² | 2,7.10⁻² |
| Témoin : nanofibrilles de cellulose non additivées et non séchées, issues de la dispersion-mère et diluées à l'Ultra-Turrax pendant une minute à 14000 tr/mn; | | | |
| Mélange 1 : 70 % de nanofibrilles et 30 % de carboxyméthylcellulose ; redispersion à la pale défloculeuse à 1000 tr/mn pendant 5 mn; | | | |
| Mélange 2 : 85 % de nanofibrilles et 15 % de carboxyméthylcellulose ; redispersion à la pale défloculeuse à 1000 tr/mn pendant 30 mn. | | | |

Il est à noter que les suspensions obtenues selon l'invention sont stables dans le temps.

On constate, par ailleurs, que l'addition de carboxymèthylcellulose à haut degré de substitution permet la redispersion de nanofibrilles séchées et de créer un état de dispersion des nanofibrilles tel, que l'on récupère, avec 15 % d'additif, au moins 72 % de la viscosité de la suspension de nanofibrilles non séchées, à un gradient de cisaillement de 1 s⁻¹, et avec 30 % d'additif au moins 134 % de la viscosité de la suspension non séchée.

En outre, le profil rhéologique de type rhéofluidifiant est conservé.

### EXEMPLE 5

### 1) Préparation des systèmes à base de nanofibrilles de cellulose, de carboxyméthylcellulose et de saccharose:

La carboxyméthylcellulose (degré de substitution égal à 1,2 ; de viscosité moyenne - produit BLANOSE 12M8P d'AQUALON) est mise en solution dans de l'eau distillée.

Le saccharose est également mis en solution dans de l'eau distillée.

La solution de carboxyméthylcellulose est ensuite ajoutée à la dispersion-mère de nanofibrilles (3,1 % en nanofibrilles de cellulose fournie par la GENERALE SUCRIERE et préhomogénéisée à l'Ultra-Turrax à 14000 tr/mn -1 mn pour 100 g de dispersion) et l'ensemble est agité à la pale défloculeuse à 1000 tr/mn pendant 30 mn.

Dans le cas de mélange sans co-additif (mélange 1), la quantité de carboxyméthylcellulose ajoutée est 15 % par rapport au poids de nanofibrilles de cellulose et de carboxyméthylcellulose. En présence du co-additif (mélange 2), la quantité de carboxyméthylcellulose ajoutée est de 10 % en poids par rapport au poids de nanofibrilles de cellulose et de carboxyméthylcellulose et de co-additif.

Le mélange est ensuite versé dans des coupelles puis séché soit dans une étuve ventilée à 40°C, jusqu'à un extrait sec de 96 %, contrôlé par dosage d'eau par la méthode KARL-FISCHER.

Le mélange séché est ensuite broyé au moulin à café, puis tamisé sur un tamis de 500 µm.

Dans le cas où la composition comprend en outre un co-additif, son addition à la dispersion-mère s'effectue en même temps que l'additif.

La solution de saccharose est alors ajoutée à la dispersion-mère de nanofibrilles déjà additivée de carboxyméthylcellulose et l'ensemble est agité à la pale défloculeuse à 1000 tr/mn pendant 30 mn.

La quantité de carboxyméthylcellulose ajoutée est de 10 % et celle de saccharose est de 20 % en poids par rapport au poids de nanofibrilles de cellulose et de carboxyméthylcellulose et de saccharose (mélange 2).

Le mélange est ensuite versé dans des coupelles puis séché soit dans une étuve ventilée à 40°C, jusqu'à un extrait sec de 96 %, contrôlé par dosage d'eau par la méthode KARL-FISCHER.

### 2) Redispersion des systèmes à base de nanofibrilles de cellulose, de carboxyméthylcellulose et de saccharose, et caractérisation :

Les poudres obtenues sont redispersées à 0,3 % en poids de nanofibrilles de cellulose dans de l'eau distillée. L'agitation s'effectue à la pale défloculeuse à 1000 tr/mn pendant 30 mn.

Une rhéologie en écoulement est effectuée au bout de 24 heures sur un rhéomètre RFS 8400 en géométrie Couette (balayage en gradient de cisaillement entre 1 et 100 s⁻¹).

Tous les systèmes sont comparés à l'échantillon témoin correspondant aux nanofibrilles de cellulose à 3,1 % en extrait sec, non séchées, et diluées dans l'eau à 0,3 % à la pale défloculeuse à 1000 tr/mn pendant 5 mn.

Mélange 1 : 85 % de nanofibrilles et 15 % de carboxyméthylcellulose ; redispersion à la pale défloculeuse à 1000 tr/mn pendant 30 mn.

Mélange 2 : 70 % de nanofibrilles, 10 % de carboxyméthylcellulose et 20 % de saccharose (co-additif); redispersion à la pale défloculeuse à 1000 tr/mn pendant 30 mn.

Le tableau III montre l'influence de la concentration en carboxyméthylcellulose ainsi que celle du co-additif, sur le profil rhéologique des nanofibrilles de cellulose après redispersion.

**Tableau III**

| **gradient de cisaillement** | **viscosité (Pa.s)** | | |
|---|---|---|---|
| **(s**^{**-1**}**)** | **témoin** | **mélange 1** | **mélange 2** |
| 1,27.10⁻¹ | 2,0 | 4,3.10⁻¹ | 2,7 |
| 2,01.10⁻¹ | 1,2 | 3,4.10⁻¹ | 1,5 |
| 5,05.10⁻¹ | 2,8.10⁻¹ | 2,4.10⁻¹ | 5,3.10⁻¹ |
| 1,27 | 9,7.10⁻² | 1,1.10⁻¹ | 2,5.10⁻¹ |
| 2,01 | 6,2.10⁻² | 7,4.10⁻² | 1,810⁻¹ |
| 5,05 | 3,5.10⁻² | 4,0.10⁻² | 7,8.10⁻² |
| 12,7 | 2,7.10⁻² | 2,6.10⁻² | 4,6.10⁻² |
| 20,1 | 1,9.10⁻² | 2,1.10⁻² | 3,8.10⁻² |
| 50,5 | 1,6.10⁻² | 1,4.10⁻² | 2,6.10⁻² |
| 80,0 | 1,3.10⁻² | 1,1.10⁻² | 2,1.10⁻² |

Il est à noter que les suspensions obtenues selon l'invention sont stables dans le temps.

On constate que l'addition de carboxyméthylcellulose seule, présentant un haut degré de substitution, permet la redispersion de nanofibrilles séchées et de créer un état de dispersion des nanofibrilles tel que l'on récupère, avec 15 % d'additif, au moins 114 % de la viscosité de la suspension de nanofibrilles non séchées, pour un gradient de cisaillement supérieur à 1 s⁻¹, et au moins 22 % de la viscosité de la suspension non séchée, pour un gradient de cisaillement voisin de 0,1 s⁻¹.

En présence de co-additif, cette récupération est de 260 % de la viscosité initiale pour un taux de cisaillement supérieur à 1 s⁻¹, et de 135 % pour un taux de cisaillement voisin de 0,1 s⁻¹. D'après ces résultats, le remplacement partiel de la carboxyméthylcellulose par du saccharose permet d'augmenter le caractère rhéofluidifiant des nanofibrilles.

### EXEMPLE 6

### 1) Préparation des systèmes à base de nanofibrilles de cellulose et de gomme xanthane:

La gomme xanthane (Rhodopol 23®) est mise en solution dans de l'eau distillée.

La solution est ensuite ajoutée à la dispersion-mère de nanofibrilles (2,9 % en nanofibrilles de cellulose fournie par la GENERALE SUCRIERE et préhomogénéisée à l'Ultra-Turrax à 14000 tr/mn (1 mn pour 100 g de dispersion)) et l'ensemble est agité à la pale défloculeuse à 1000 tr/mn pendant 30 mn.

La quantité de gomme xanthane ajoutée est de 30 % en poids, par rapport au poids de nanofibrilles de cellulose et de gomme xanthane.

Le mélange est ensuite versé dans des coupelles puis séché soit dans une étuve ventilée à 40°C, jusqu'à un extrait sec de 97 %, contrôlé par dosage d'eau par la méthode KARL-FISCHER.

Le mélange séché est ensuite broyé au moulin à café, puis tamisé sur un tamis de 500 µm.

### 2) Redispersion des systèmes à base de nanofibrilles de cellulose et de gomme xanthane, et caractérisation :

La poudre obtenue est redispersée à 0,3 % en poids de nanofibrilles de cellulose dans de l'eau distillée. L'agitation s'effectue à la pale défloculeuse à 1000 tr/mn pendant 30 mn.

Une rhéologie en écoulement est effectuée au bout de 24 heures sur un rhéomètre RFS 8400 en géométrie Couette (balayage en gradient de cisaillement entre 1 et 100 s⁻¹).

Tous les systèmes sont comparés aux nanofibrilles de cellulose non séchées et diluées dans l'eau à 0,3 % à l'Ultra-Turrax à 14000 tr/mn pendant 1 mn (état de redispersion optimal des nanofibrilles).

Le tableau IV montre l'effet de la gomme xanthane sur le profil rhéologique des nanofibrilles de cellulose après redispersion.

**Tableau IV**

| **gradient de cisaillement** **(s**^{**-1**}**)** | **viscosité (Pa.s)** | |
|---|---|---|
| | **témoin** | **mélange 1** |
| 1,27 | 4,1.10⁻¹ | 10,0.10⁻¹ |
| 2,01 | 2,6.10⁻¹ | 6,0.10⁻¹ |
| 5,05 | 1,3.10⁻¹ | 3,0.10⁻¹ |
| 12,7 | 1,0.10⁻¹ | 1,5.10⁻¹ |
| 20,1 | 6,0.10⁻² | 1,0.10⁻¹ |
| 50,5 | 2,8.10⁻² | 5,0.10⁻² |
| 80,0 | 2,5.10⁻² | 3,8.10⁻² |
| Témoin : nanofibrilles de cellulose non additivées et non séchées, issues de la dispersion-mère et diluées à l'Ultra-Turrax pendant une minute à 14000 tr/mn; | | |
| Mélange 1 : 70 % de nanofibrilles et 30 % de gomme xanthane ; redispersion à la pale défloculeuse à 1000 tr/mn pendant 30 mn; | | |

Il est à noter que la suspension obtenue selon l'invention est stable dans le temps.

On constate, par ailleurs, que l'addition de gomme xanthane permet la redispersion de nanofibrilles séchées et de créer un état de dispersion des nanofibrilles tel, que l'on récupère, avec 30 % d'additif, au moins 240 % de la viscosité de la suspension de nanofibrilles non séchées, à un gradient de cisaillement de 1 s⁻¹.

En outre, le profil rhéologique de type rhéofluidifiant est conservé.

## Revendications

1. Composition comprenant des nanofibrilles de cellulose essentiellement amorphes, présentant un taux de cristallinité inférieur ou égal à 50 %, au moins un additif choisi parmi la cellulose carboxylée présentant un degré de substitution supérieur à 0,95, et éventuellement au moins un co-additif, la teneur en additif et en co-additif éventuel étant inférieure ou égale à 30 % en poids par rapport au poids de nanofibrilles et d'additif et de co-additif éventuel.

2. Composition selon la revendication 1, **caractérisée en ce que** les nanofibrilles présentent un taux de cristallinité compris entre 15 % et 50 %.

3. Composition selon l'une des revendications 1 ou 2, **caractérisée en ce que** les nanofibrilles de cellulose sont chargées en acides et en polysaccharides acides, seuls ou en mélange.

4. Composition selon l'une quelconque des revendications 1 à 3, **caractérisée en ce qu'**elle comprend au moins un co-additif choisi parmi :
- la cellulose carboxylée présentant un degré de substitution inférieur ou égal à 0,95, de préférence la cellulose carboxyméthylée,
- les monomères ou oligomères osidiques,
- les composés de formule (R¹R²N)COA, formule dans laquelle R¹ ou R², identiques ou différents, représentent l'hydrogène ou un radical alkyle en C₁-C₁₀, de préférence en C₁-C₅, A représente l'hydrogène, un radical alkyle en C₁-C₁₀, de préférence en C₁-C₅, ou encore le groupement R'¹R'²N avec R'¹, R'², identiques ou différents, représentant l'hydrogène ou un radical alkyle en C₁-C₁₀, de préférence en C₁-C₅,
- les tensioactifs cationiques ou amphotères,
ces co-additifs pouvant être utilisés seuls ou en mélange.

5. Composition selon l'une quelconque des revendications précédentes, **caractérisée en ce que** la teneur en co-additif est inférieure à 30 % en poids par rapport au poids de nanofibrilles et d'additif et de co-additif.

6. Composition selon l'une quelconque des revendications précédentes, **caractérisée en ce que** le co-additif est choisi parmi les monomères ou oligomères osidiques, la cellulose carboxylée présentant un faible degré de substitution qui est inférieur ou égale à 0,95 ou les composés de formule (R¹R²N)COA avec une teneur inférieure à 30 % et de préférence comprise entre 1 et 25 % en poids par rapport au poids de nanofibrilles et d'additif et de co-additif.

7. Composition selon l'une quelconque des revendications précédentes, **caractérisée en ce que** le co-additif est choisi parmi les tensioactifs cationiques ou amphotères avec une teneur comprise entre 1 et 10 % en poids par rapport au poids de nanofibrilles et d'additif et de co-additif.

8. Composition selon l'une quelconque des revendications précédentes, **caractérisée en ce que** la teneur en additif et en co-additif est inférieure ou égale à 25 % par rapport au poids de nanofibrilles et d'additif et de co-additif, de préférence encore comprise entre 5 et 25 % par rapport à la même référence.

9. Composition selon l'une quelconque des revendications précédentes, **caractérisée en ce que** la teneur en matières sèches est d'au moins 40 % en poids.

10. Procédé de préparation d'une composition selon l'une quelconque des revendications 1 à 9 dans lequel on prépare des nanofibrilles de cellulose à partir de pulpe cellulosique en effectuant au moins une extraction, éventuellement au moins une étape de blanchiment de la pulpe ainsi traitée, puis on sépare la pulpe résultante, et l'on met en oeuvre une étape d'homogénéisation en au moins un cycle, **caractérisé en ce que** l'on effectue les étapes suivantes :
- on ajoute à la suspension de nanofibrilles ayant éventuellement subi au moins un cycle d'homogénéisation, au moins une partie de l'additif et éventuellement du ou des co-additifs,
- on effectue une étape de séchage de la suspension ainsi additivée.

11. Procédé de préparation selon la revendication précédente, **caractérisé en ce que** l'on ajoute au moins une partie de l'additif et éventuellement du ou des co-additifs, à la suspension à l'issue de l'étape d'homogénéisation.

12. Procédé de préparation selon la revendication précédente, **caractérisé en ce que** l'on ajoute au moins une partie de l'additif et éventuellement du ou des co-additifs, à la suspension à l'issue de l'étape d'homogénéisation, après que cette dernière a subi au moins une étape de concentration.

13. Procédé de préparation selon la revendication 11, **caractérisé en ce que** l'on ajoute au moins une partie de l'additif et éventuellement du ou des co-additifs, à la suspension à l'issue de l'étape d'homogénéisation, avant que ladite suspension ait subi une étape de concentration.

14. Procédé de préparation selon l'une quelconque les revendications 12 ou 13, **caractérisé en ce que** l'on effectue l'étape de concentration pour obtenir une suspension présentant une teneur en extrait sec d'environ au plus 35 % en poids.

15. Procédé de préparation selon la revendication 11, **caractérisé en ce que** l'on ajoute au moins une partie de l'additif et éventuellement du ou des co-additifs, à la suspension avant ou pendant l'étape d'homogénéisation.

16. Procédé de préparation selon l'une quelconque des revendications 10 à 15, **caractérisé en ce que** préalablement au séchage, on effectue une mise en forme de la suspension de nanofibrilles de cellulose.

17. Procédé de préparation selon l'une quelconque des revendications 10 à 16, **caractérisé en ce que** l'on effectue l'étape de séchage de manière à maintenir au minimum 3 % en poids d'eau par rapport au poids du solide obtenu.

18. Procédé de préparation selon l'une quelconque des revendications 10 à 16, **caractérisé en ce que** l'on effectue l'étape de séchage de manière à maintenir au minimum 5 % en poids d'eau par rapport au poids de nanofibrilles de cellulose.

19. Procédé de préparation selon l'une quelconque des revendications 10 à 17, **caractérisé en ce que** l'on effectue l'étape de séchage de manière à maintenir le poids d'eau entre 10 et 30 % en poids.

20. Procédé de préparation selon la revendication précédente, **caractérisé en ce que** l'on met en oeuvre une étape de broyage à l'issue du séchage.

21. Suspension comprenant des nanofibrilles de cellulose, **caractérisée en ce qu'**elle est obtenue en dispersant la composition selon l'une quelconque des revendications 1 à 9 ou obtenue selon l'une quelconque des revendications 10 à 20.

22. Suspension selon la revendication précédente, **caractérisée en ce qu'**elle présente un profil rhéologique de type rhéofluidifiant.

23. Suspension selon l'une quelconque des revendications 21 ou 22, **caractérisée en ce qu'**elle présente un niveau de viscosité correspondant à au moins 50 % pour un taux de cisaillement d'au moins 1 s⁻¹, du niveau de viscosité d'une suspension de nanofibrilies de cellulose n'ayant pas subi d'étape de séchage et ne comprenant pas d'additif, ni de co-additifs.

24. Utilisation de cellulose carboxylée présentant un degré de substitution supérieur à 0,95, et éventuellement au moins un co-additif, avec des nanofibrilles de cellulose essentiellement amorphes, présentant un taux de cristallinité inférieur ou égal à 50 %, dans le but de conserver un profil rhéologique rhéofluidifiant à une suspension comprenant des nanofibrilles de cellulose essentiellement amorphes ayant subi une étape de séchage.

25. Utilisation des compositions selon l'une quelconque des revendications 1 à 9, ainsi que des suspensions selon l'une quelconque des revendications 21 à 23 comme additif dans des formulations destinées au domaine de la cosmétique et / ou de la détergence.

26. Utilisation des compositions selon l'une quelconque des revendications 1 à 9, ainsi que des suspensions selon l'une quelconque des revendications 21 à 23 comme additif dans des formulations alimentaires.

27. Utilisation des compositions selon l'une quelconque des revendications 1 à 9, ainsi que des suspensions selon l'une quelconque des revendications 21 à 23 comme additif dans des formulations pour les travaux publics et le bâtiment.

## Patentansprüche

1. Zusammensetzung von Nanofibrillen von im wesentlichen amorpher Cellulose mit einem Kristallinitätsgrad von unter oder gleich 50 %, mindestens einem Zusatzstoff, ausgewählt unter carboxylierter Cellulose mit einem Substitutionsgrad von über 0,95 und gegebenenfalls mindestens einem Co-Zusatzstoff, wobei der Gehalt an Zusatzstoff und an eventuellem Co-Zusatzstoff unter oder gleich 30 Gew.-% beträgt, bezogen auf das Gewicht von Nanofibrillen und Zusatzstoff und eventuellem Co-Zusatzstoff.

2. Zusammensetzung nach Anspruch 1, **dadurch gekennzeichnet, daß** die Nanofibrillen einen Kristallinitätsgrad zwischen 15 % und 50 % aufweisen.

3. Zusammensetzung nach einem der Ansprüche 1 oder 2, **dadurch gekennzeichnet, daß** die Nanofibrillen von Cellulose mit Säuren und sauren Polysacchariden, allein oder in Mischung, beladen sind.

4. Zusammensetzung nach irgendeinem der Ansprüche 1 bis 3, **dadurch gekennzeichnet, daß** sie mindestens einen Co-Zusatzstoff umfaßt, ausgewählt unter:
- carboxylierter Cellulose mit einem Substitutionsgrad von unter oder gleich 0,95, vorzugsweise Carboxymethylcellulose,
- den osidischen Monomeren oder Oligomeren,
- den Verbindungen der Formel (R¹R²N)COA, worin R¹ oder R², gleich oder verschieden, Wasserstoff oder einen Rest Alkyl mit 1 bis 10 Kohlenstoffatomen, vorzugsweise 1 bis 5 Kohlenstoffatomen darstellen, A Wasserstoff, einen Rest Alkyl mit 1 bis 10 Kohlenstoffatomen, vorzugsweise 1 bis 5 Kohlenstoffatomen, oder auch die Gruppe R'¹R'²N bedeutet, worin R'¹, R'², gleich oder verschieden, Wasserstoff oder einen Rest Alkyl mit 1 bis 10 Kohlenstoffatomen, vorzugsweise 1 bis 5 Kohlenstoffatomen, darstellen,
- den kationischen oder amphoteren oberflächenaktiven Mitteln, wobei diese Co-Zusatzstoffe allein oder in Mischung verwendet werden können.

5. Zusammensetzung nach irgendeinem der vorstehenden Ansprüche, **dadurch gekennzeichnet, daß** der Gehalt an Co-Zusatzstoff unter 30 Gew.-% beträgt, bezogen auf das Gewicht von Nanofibrillen und Zusatzstoff und Co-Zusatzstoff.

6. Zusammensetzung nach irgendeinem der vorstehenden Ansprüche, **dadurch gekennzeichnet, daß** der Co-Zusatzstoff ausgewählt wird unter den osidischen Monomeren oder Oligomeren, der carboxylierten Cellulose mit einem niedrigen Substitutionsgrad von unter oder gleich 0,95 oder den Verbindungen der Formel (R¹R²N)COA mit einem Gehalt von unter 30 % und vorzugsweise zwischen 1 und 25 Gew.-%, bezogen auf das Gewicht von Nanofibrillen und Zusatzstoff und Co-Zusatzstoff.

7. Zusammensetzung nach irgendeinem der vorstehenden Ansprüche, **dadurch gekennzeichnet, daß** der Co-Zusatzstoff ausgewählt wird unter den kationischen oder amphoteren oberflächenaktiven Mitteln mit einem Gehalt zwischen 1 und 10 Gew.-%, bezogen auf das Gewicht von Nanofibrillen und Zusatzstoff und Co-Zusatzstoff.

8. Zusammensetzung nach irgendeinem der vorstehenden Ansprüche, **dadurch gekennzeichnet, daß** der Gehalt an Zusatzstoff und an Co-Zusatzstoff unter oder gleich 25 % beträgt, bezogen auf das Gewicht von Nanofibrillen und Zusatzstoff und Co-Zusatzstoff, noch bevorzugter zwischen 5 und 25 %, bezogen auf die gleiche Referenz.

9. Zusammensetzung nach irgendeinem der vorstehenden Ansprüche, **dadurch gekennzeichnet, daß** der Gehalt an Trockenmasse mindestens 40 Gew.-% beträgt.

10. Verfahren zur Herstellung einer Zusammensetzung nach irgendeinem der Ansprüche 1 bis 9, bei dem man Nanofibrillen von Cellulose ausgehend von cellulosischer Pulpe herstellt, indem man mindestens eine Extraktion, gegebenenfalls mindestens eine Stufe des Bleichens der auf diese Weise behandelten Pulpe durchführt, anschließend die resultierende Pulpe abtrennt und man eine Stufe der Homogenisierung in mindestens einem Zyklus durchführt, **dadurch gekennzeichnet, daß** man die folgenden Stufen vornimmt:
- man gibt zu der Suspension von Nanofibrillen, die gegebenenfalls mindestens einem Zyklus der Homogenisierung unterzogen wurde, mindestens einen Teil des Zusatzstoffes und gegebenenfalls den oder die Co-Zusatzstoff(e),
- man führt eine Stufe der Trocknung der auf diese Weise mit Zusatzstoff versehenen Suspension durch.

11. Verfahren zur Herstellung nach vorstehendem Anspruch, **dadurch gekennzeichnet, daß** man mindestens einen Teil des Zusatzstoffes und gegebenenfalls den oder die Co-Zusatzstoff(e) zu der Suspension am Ende der Stufe der Homogenisierung gibt.

12. Verfahren zur Herstellung nach vorstehendem Anspruch, **dadurch gekennzeichnet, daß** man mindestens einen Teil des Zusatzstoffes und gegebenenfalls den oder die Co-Zusatzstoff(e) zu der Suspension am Ende der Stufe der Homogenisierung gibt, nachdem diese letztere mindestens einer Stufe der Konzentration unterzogen wurde.

13. Verfahren zur Herstellung nach Anspruch 11, **dadurch gekennzeichnet, daß** man mindestens einen Teil des Zusatzstoffes und gegebenenfalls den oder die Co-Zusatzstoff(e) zu der Suspension am Ende der Stufe der Homogenisierung gibt, bevor die genannte Suspension einer Stufe der Konzentration unterzogen wurde.

14. Verfahren zur Herstellung nach irgendeinem der Ansprüche 12 oder 13, **dadurch gekennzeichnet, daß** man die Stufe der Konzentration durchführt, um eine Suspension mit einem Gehalt an Trockenextrakt von höchstens etwa 35 Gew.-% zu erhalten.

15. Verfahren zur Herstellung nach Anspruch 11 , **dadurch gekennzeichnet, daß** man mindestens einen Teil des Zusatzstoffes und gegebenenfalls den oder die Co-Zusatzstoff(e) zu der Suspension vor oder während der Stufe der Homogenisierung gibt.

16. Verfahren zur Herstellung nach irgendeinem der Ansprüche 10 bis 15, **dadurch gekennzeichnet, daß** man vor der Trocknung eine Formgebung der Suspension von Cellulose-Nanofibrillen durchführt.

17. Verfahren zur Herstellung nach irgendeinem der Ansprüche 10 bis 16, **dadurch gekennzeichnet, daß** man die Stufe der Trocknung in der Weise durchführt, daß man im Minimum 3 Gew.-% Wasser aufrechterhält, bezogen auf das Gewicht der Cellulose-Nanofibrillen.

18. Verfahren zur Herstellung nach irgendeinem der Ansprüche 10 bis 16, **dadurch gekennzeichnet, daß** man die Stufe der Trocknung in der Weise durchführt, daß man im Minimum 5 Gew.-% Wasser aufrechterhält, bezogen auf das Gewicht der Cellulose-Nanofibrillen.

19. Verfahren zur Herstellung nach irgendeinem der Ansprüche 10 bis 17, **dadurch gekennzeichnet, daß** man die Stufe der Trocknung in der Weise durchführt, daß man das Gewicht von Wasser zwischen 10 und 30 Gew-% hält.

20. Verfahren zur Herstellung nach dem vorstehenden Anspruch, **dadurch gekennzeichnet, daß** man am Ende der Trocknung eine Stufe der Zerkleinerung durchführt.

21. Suspension von Cellulose-Nanofibrillen, **dadurch gekennzeichnet, daß** sie erhalten wird, indem man die Zusammensetzung nach irgendeinem der Ansprüche 1 bis 9 oder wie sie nach irgendeinem der Ansprüche 10 bis 20 erhalten wird, suspendiert.

22. Suspension nach dem vorstehenden Anspruch, **dadurch gekennzeichnet, daß** sie ein rheologisches Profil vom rheofluidisierenden Typ aufweist.

23. Suspension nach irgendeinem der Ansprüche 21 oder 22, **dadurch gekennzeichnet, daß** sie ein Viskositätsniveau aufweist, das mindestens 50 % bei einem Schergrad von mindestens 1 s⁻¹ von dem Viskositätsniveau einer Suspension von Cellulose-Nanofibrillen entspricht, die nicht einer Stufe der Trocknung unterzogen wurde und die weder Zusatzstoff noch Co-Zusatzstoff umfaßt.

24. Verwendung von carboxylierter Cellulose mit einem Substitutionsgrad von über 0,95 und gegebenenfalls mindestens einem Co-Zusatzstoff mit im wesentlichen amorphen Nanofibrillen von Cellulose, die einen Kristallinitätsgrad von unter oder gleich 50 % aufweisen, mit dem Ziel, ein rheologisches rheofluidisierendes Profil bei einer Suspension aufrechtzuerhalten, die im wesentlichen amorphe Nanofibrillen von Cellulose umfaßt, die ihrerseits einer Stufe der Trocknung unterzogen wurden.

25. Verwendung der Zusammensetzungen nach irgendeinem der Ansprüche 1 bis 9 sowie der Suspensionen nach irgendeinem der Ansprüche 21 bis 23 als Zusatzstoff in Formulierungen, die für das Gebiet der Kosmetik und/oder der Reinigungsmittel vorgesehen sind.

26. Verwendung der Zusammensetzungen nach irgendeinem der Ansprüche 1 bis 9 sowie der Suspensionen nach irgendeinem der Ansprüche 21 bis 23 als Zusatzstoff in Nahrungsmittel-Formulierungen.

27. Verwendung der Zusammensetzungen nach irgendeinem der Ansprüche 1 bis 9 sowie der Suspensionen nach irgendeinem der Ansprüche 21 bis 23 als Zusatzstoff in Formulierungen für den Tiefbau und das Bauwesen.

## Claims

1. A composition comprising essentially amorphous cellulose nanofibrils having a degree of crystallinity of less than or equal to 50%, at least one additive selected from carboxylated cellulose having a degree of substitution of greater than 0.95, and optionally at least one co-additive, the proportion of additive and optional co-additive being less than or equal to 30% by weight with respect to the weight of nanofibrils and additive and optional co-additive.

2. A composition according to claim 1 **characterised in that** the nanofibrils have a degree of crystallinity of between 15% and 50%.

3. A composition according to one of claims 1 and 2 **characterised in that** the cellulose nanofibrils are charged with acids and acid polysaccharides, alone or in the form of a mixture.

4. A composition according to any one of claims 1 to 3 **characterised in that** it comprises at least one co-additive selected from:
- carboxylated cellulose having a degree of substitution of less than or equal to 0.95, preferably carboxymethylated cellulose,
- osidic monomers or oligomers,
- compounds of the formula (R¹R²N)COA, in which formula R¹ or R² which are identical or different represent hydrogen or a C₁-C₁₀, preferably C₁-C₅ alkyl radical, A represents hydrogen, a C₁-C₁₀, preferably C₁-C₅ alkyl radical, or the grouping R'¹R'²N with R'¹, R'², which are identical or different, representing hydrogen or a C₁-C₁₀, preferably C₁-C₅ alkyl radical,
- cationic or amphoteric surfactants,
wherein said co-additives can be used alone or in the form of a mixture.

5. A composition according to any one of the preceding claims **characterised in that** the amount of co-additive is less than 30% by weight with respect to the weight of nanofibrils and additive and co-additive.

6. A composition according to any one of the preceding claims **characterised in that** the co-additive is selected from osidic monomers or oligomers, the carboxylated cellulose having a low degree of substitution which is less than or equal to 0.95 or the compounds of the formula (R¹R²N)COA with a proportion of less than 30% and preferably between 1 and 25% by weight with respect to the weight of nanofibrils and additive and co-additive.

7. A composition according to one of the preceding claims **characterised in that** the co-additive is selected from cationic or amphoteric surfactants with a proportion of between 1 and 10% by weight with respect to the weight of nanofibrils and additive and co-additive.

8. A composition according to any one of the preceding claims **characterised in that** the proportion of additive and co-additive is less than or equal to 25% with respect to the weight of nanofibrils and additive and co-additive, preferably again between 5 and 25% by weight with respect to the same reference.

9. A composition according to any one of the preceding claims **characterised in that** the proportion of dry materials is at least 40% by weight.

10. A process for the preparation of a composition according to any one of claims 1 to 9 wherein cellulose nanofibrils are prepared from cellulose pulp by effecting at least one extraction operation, optionally at least one step for bleaching the pulp treated in that way, then the resulting pulp is separated, and a homogenisation step is effected in at least one cycle, **characterised in that** the following steps are effected:
- at least a part of the additive and optionally the co-additive or co-additives is added to the suspension of nanofibrils which has possibly been subjected to at least one homogenisation cycle, and
- a step for drying the suspension to which additives have been added **in that** way is effected.

11. A preparation process according to the preceding claim **characterised in that** at least a part of the additive and optionally the co-additive or co-additives is added to the suspension at the end of the homogenisation step.

12. A preparation process according to the preceding claim **characterised in that** at least a part of the additive and optionally the co-additive or co-additives is added to the suspension at the end of the homogenisation step after the latter has been subjected to at least one concentration step.

13. A preparation process according to the preceding claim **characterised in that** at least a part of the additive and optionally the co-additive or co-additives is added to the suspension at the end of the homogenisation step before said suspension has been subjected to a concentration step.

14. A preparation process according to either one of claims 12 and 13 **characterised in that** the concentration step is effected to obtain a suspension having a proportion of dry extract of about at most 35% by weight.

15. A preparation process according to claim 11 **characterised in that** at least a part of the additive and optionally the co-additive or co-additives is added to the suspension prior to or during the homogenisation step.

16. A preparation process according to any one of claims 10 to 15 **characterised in that** prior to the drying operation an operation for shaping the cellulose nanofibril suspension is effected.

17. A preparation process according to any one of claims 10 to 16 **characterised in that** the drying step is carried out in such a way as to maintain at the minimum 3% by weight of water with respect to the weight of cellulose nanofibrils.

18. A preparation process according to any one of claims 10 to 16 **characterised in that** the drying step is carried out in such a way as to maintain at the minimum 5% by weight of water with respect to the weight of cellulose nanofibrils.

19. A preparation process according to any one of claims 10 to 17 **characterised in that** the drying step is carried out in such a way as to maintain the weight of water at between 10 and 30% by weight.

20. A preparation process according to the preceding claim **characterised in that** a crushing step is effected at the end of the drying operation.

21. A suspension comprising cellulose nanofibrils **characterised in that** it is obtained by dispersing the composition according to any one of claims 1 to 9 or obtained according to any one of claims 10 to 20.

22. A suspension according to the preceding claim **characterised in that** it has a rheological profile of rheofluidifying type.

23. A suspension according to either one of claims 21 and 22 **characterised in that** it has a level of viscosity corresponding to at least 50% for a shearing rate of at least 1 s⁻¹ of the level of viscosity of a cellulose nanofibril suspension which has not been subjected to any drying step and not comprising additive or co-additive.

24. Use of carboxylated cellulose having a degree of substitution of greater than 0.95 and optionally at least one co-additive with essentially amorphous cellulose nanofibrils having a degree of crystallinity of less than or equal to 50% for the purposes of preserving a rheofluidifying rheological profile for a suspension comprising essentially amorphous cellulose nanofibrils which has been subjected to a drying step.

25. Use of the compositions according to any one of claims 1 to 9 and the suspensions according to any one of claims 21 to 23 as an additive in formulations intended for the sector of cosmetics and/or detergence.

26. Use of the compositions according to any one of claims 1 to 9 and the suspensions according to any one of claims 21 to 23 as an additive in foodstuff formulations.

27. Use of the compositions according to any one of claims 1 to 9 and the suspensions according to any one of claims 21 to 23 as an additive in formulations for public works and building.
